# EUROPEAN PATENT SPECIFICATION

(11) **EP 1 474 436 B1**
(45) Date of publication and mention of the grant of the patent: **28.10.2009**
(21) Application number: 03704541.6
(22) Date of filing: 03.02.2003
(51) Int. Cl.: C07J 31/00

(54) **PROCESS FOR THE PRODUCTION OF FLUTICASONE PROPIONATE, IN PARTICULAR OF POLYMORPHIC FORM 1**
VERFAHREN ZUR HERSTELLUNG VON FLUTICASONE PROPIONATEN, INSBESONDERE POLYMORPH 1
PROCEDE POUR LA PREPARATION DU PROPIONATE DE FLUTICASONE,NOTAMMENT DE LA FORME POLYMORPHE 1

(30) Priority: 04.02.2002 GB 0202564
(43) Date of publication of application: 10.11.2004
(73) Proprietor: GLAXO GROUP LIMITED, Greenford, Middlesex UB6 0NN (GB)
(72) Inventor: Coote, Steven John, Stevenage, Hertfordshire SG1 2NY (GB); Nice, Rosalyn Kay, Stevenage, Hertfordshire SG1 2NY (GB); Wipperman, Mark David, Stevenage, Hertfordshire SG1 2NY (GB)
(74) Representative: Povey, Alexander W.G.
(86) International application number: PCT/EP2003/001115
(87) International publication number: WO 2003/066653

(56) References cited:
- WO-A-01/32125
- WO-A-02/12265
- WO-A-98/17676
- PHILLIPPS G H ET AL: "SYNTHESIS AND STRUCTURE-ACTIVITY RELATIONSHIPS IN A SERIES OF ANTIINFLAMMATORY CORTICOSTEROID ANALOGUES, HALOMETHYL ANDROSTANE 17BETA-CARBOTHIOATES AND -17BETA-CARBOSELENOATES" JOURNAL OF MEDICINAL CHEMISTRY, AMERICAN CHEMICAL SOCIETY. WASHINGTON, US, vol. 37, no. 22, 28 October 1994 (1994-10-28), pages 3717-3729, XP002052064 ISSN: 0022-2623 cited in the application
- KARIUKI, BENSON M. ET AL: "Structure determination of a steroid directly from powder diffraction data" CHEMICAL COMMUNICATIONS (CAMBRIDGE) (1999), (17), 1677-1678 , XP002251718

## Description

This invention relates to a novel process for preparing fluticasone propionate, especially fluticasone propionate as crystalline Form 1 polymorph.

Fluticasone propionate is a corticosteroid of the androstane family which has potent anti-inflammatory activity and is widely accepted as a useful therapy for the treatment of inflammatory and allergic conditions such as rhinitis, asthma and chronic obstructive pulmonary disease (COPD). The chemical representation of fluticasone propionate is as shown in the structure below:

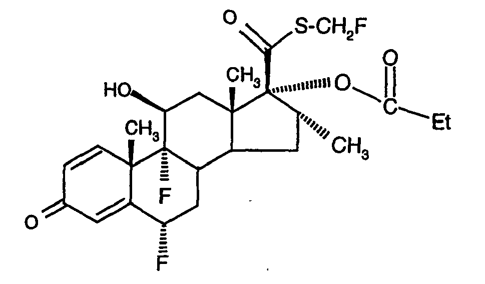

Fluticasone propionate and methods for preparing it were first described in British Patent 2088877 (see Example 14 thereof). A crystalline form of fluticasone propionate, designated polymorphic Form 1, may be obtained by dissolving the crude product (obtained eg as described in GB 2088877) in ethyl acetate and then recrystallising. Standard spray-drying processes have also been shown to lead to the Form 1 of fluticasone propionate. Phillips et al (1994) J Med Chem, 37, 3717-3729 also describes the direct recrystallisation of fluticasone propionate from acetone. International Patent Application WO98/17676 disclosed a new polymorphic form of fluticasone propionate, designated polymorphic Form 2, which could be obtained by supercritical fluid techniques. Modification of the supercritical conditions could also lead to generation of the Form 1 polymorph. Example conditions described in WO98/17676 for preparing Form 1 included co-introducing carbon dioxide and a solution of fluticasone propionate dissolved in acetone via a co-axial nozzle into a particle generation vessel. Polymorphic Forms 1 and 2 of fluticasone propionate may be distinguished by their XRPD profiles and unit cell dimensions. For example crystalline polymorphic Form 1 has a monoclinic crystal structure with characteristics as follows:
a = 7.722', b = 14.176', c = 11.290', β = 98.458°

By contrast, crystalline Polymorphic Form 2 has an orthorhombic crystal structure with characteristics as follows:
a = 23.404', b = 14.048', c = 7.695', all angles = 90°

Characteristic peaks in the XRPD profiles are given in the following table:

| Polymorph | Primary peaks (°2Theta) | Secondary peaks (°2Theta) |
|---|---|---|
| Form 1 | 7.9, 10.0 | 11.5, 12.4, 13.1, -. 14.9, -, 15.8 |
| Form 2 | 7.6, 9.8 | -, -, 13.0, 13.6, -. 15.2, - |

The XRPD profiles of the two polymorphs are shown in Figure 1.

International Patent Application WO00/38811 (Glaxo Group) discloses the crystallisation of fluticasone propionate dissolved in acetone by mixing with water in the presence of ultrasound radiation. International Patent Application WO01/32125 (Glaxo Group) discloses the crystallisation of fluticasone propionate by admitting a stream of solution of fluticasone propionate in acetone and a stream of water as anti-solvent tangentially into a cylindrical mixing chamber having an axial outlet port such that said streams are thereby intimately mixed through formation of a vortex. We have now invented a new method for preparing fluticasone propionate as crystalline polymorphic Form 1.

Thus according to the invention there is provided a process (hereinafter referred to as "main process") for preparing fluticasone propionate as crystalline polymorphic Form 1 which comprises mixing a solution of fluticasone propionate in a non-solvating organic liquid solvent with a non-solvating organic liquid anti-solvent thereby causing fluticasone propionate as crystalline polymorphic Form 1 to crystallise out of the solution.

The process is performed at ambient atmospheric pressure such that the solvent and anti-solvent are liquids at ambient atmospheric pressure.

The non-solvating organic liquid solvent and non-solvating organic liquid anti-solvent will be liquids which do not have a tendency to form crystalline solvates with fluticasone propionate. The non-solvating organic liquid solvent is selected from methyl acetate, ethyl acetate and pentanone, for example pentan-3-one, especially ethyl acetate, more especially pentan-3-one. The non-solvating organic liquid anti-solvent is selected from toluene, isooctane or hexane, particularly hexane, more particularly toluene. By definition water is not an organic liquid anti-solvent.

The process according to the invention is advantageous over prior art processes, for example: it can be performed at ambient pressure without need for use of elaborate apparatus eg to enable use of supercritical carbon dioxide, or use of ultrasound, or to create vortex mixing conditions. Also in one aspect of the invention it permits fluticasone propionate to be prepared as crystalline polymorph Form 1 directly from precursors of fluticasone propionate without need for fluticasone propionate in subtantially pure form to be isolated as an intermediate step in the process. Thus it has economic and other processing advantages since the number of steps in the overall synthesis may be reduced.

One process for preparing fluticasone propionate (hereinafter referred to as "process (a)") comprises reacting a compound of formula (II) 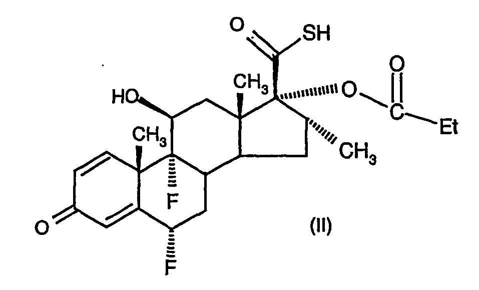 or a salt thereof
with a compound of formula LCH₂F, where L represents a leaving group mesyl, tosyl or halogen, eg Cl, Br or I. Preferably L represents halogen, particularly Br.

Advantageous conditions for performing this step comprise combining the compound of formula (II) or a salt thereof optionally in the presence of a phase-transfer catalyst, a water-immiscible non-solvating organic solvent and water. When a free acid of compound of formula (II) is employed, then a base should also be included to remove the -SH proton. Suitable bases include tri-n-propylamine, tributylamine, sodium bicarbonate, triethylamine, diisopropylethylamine and the like.

Preferably the water immiscible non-solvating organic solvent is a non-solvating organic solvent selected from the list mentioned above. So as to benefit from the advantages of process (a) the non-solvating liquid organic solvent used in the main process is preferably the same solvent i.e. it is immiscible with water, and is most preferably ethyl acetate, even moreso, pentan-3-one. This avoids the need for an isolation step.

As used herein, substantially immiscible solvents provide two phases when the solvents are mixed and have a low level of solubility of one in the other eg the solubility of one solvent in the other solvent is less than 10% w/w, especially 5% w/w. Examples of phase-transfer catalysts that may be employed include tetrabutylammonium bromide, tetrabutylammonium chloride, benzyltributylammonium bromide, benzyltributylammonium chloride, benzyltriethylammonium bromide, methyltributylammonium chloride and methyltrioctylammonium chloride, preferably benzyltributylammonium chloride, benzyltriethylammonium bromide, especially benzyltributylammonium chloride.

Preferably the phase-transfer catalyst will be employed in an amount of 1-15mol% based on compound of formula (II), especially 5-12%, particularly around 10%.

The advantages of this process include the fact that the presence of a phase transfer catalyst in the reaction mixture results in a significantly faster reaction rate relative to its absence. Other advantages of the invention include the fact that fluticasone propionate as crystalline polymorph Form 1 may be isolated directly from the alkylation solvent without need for an intermediate isolation step.

A further advantage is that the use of methyl ethyl ketone or addition of methyl ethyl ketone to ethyl acetate increases the rate of reaction.

The fluticasone propionate product present in an organic phase following process (a) is preferably increased in purity by washing firstly with aqueous acid eg dilute HCl in order to remove amine compounds such as triethylamine and diisopropylethylamine (which may be present following process (b) described below) and then with aqueous base eg sodium bicarbonate in order to remove any unreacted precursor compound of formula (II). The washed organic layer (which may be separated from the more dense aqueous layer by conventional means, eg by running it off) is preferably concentrated by distillation (optionally under reduced pressure) before being treated with the organic anti-solvent according to the main process described above.

Thus the invention provides a process for preparing fluticasone propionate as crystalline polymorphic Form 1 which comprises
(a) reacting a compound of formula (II) 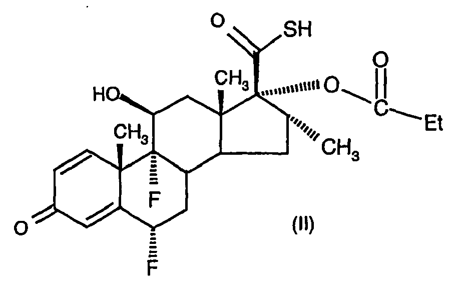 or a salt thereof with a compound of formula LCH₂F wherein L represents leaving group optionally in the presence of a phase transfer catalyst, a water-immiscible non-solvating organic liquid solvent and water;
(b) optionally increasing the purity of the fluticasone propionate in the organic layer by performing one or more aqueous washing steps;
(c) separating the organic layer from the aqueous layer and optionally concentrating the fluticasone propionate in the organic layer;
(d) mixing the solution of fluticasone propionate in the water-immiscible non-solvating organic liquid solvent present in the organic layer with a non-solvating organic liquid anti-solvent thereby causing fluticasone propionate as crystalline polymorphic Form 1 to crystallise out of the solution.

According to prior art processes, eg as described in G.H. Phillips et al (1994) J Med Chem 37, 3717-3729*,* US patent 4,335,121 (Glaxo Group Limited), compounds of formula (II) may be prepared by reacting a compound of formula (III) 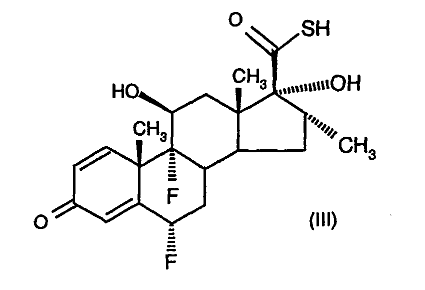 with an activated derivative of propionic acid eg propionyl chloride. The activated derivative of propionic acid will generally be employed in at least 2 times molar quantity relative to the compound of formula (III) since one mole of the reagent will react with the thioacid moiety and needs to be removed eg by reaction with an amine such as diethylamine. This method for the preparation of compound of formula (II) suffers from disadvantages, however, in that the resultant compound of formula (II) is not readily purified of contamination with the by-product N,N-diethylpropanamide. We have therefore provided an improved process for performing the conversion to prepare compound of formula (II). Other processes for preparation of fluticasone propionate and related compounds are described in Israeli patent application 109656 Chemagis), WO01/62722 (Abbott) and Kertesz and Marx (1986) J Org Chem 51, 2315-2328*.*

Thus there is provided a process (hereinafter "process (b")) for preparing a compound of formula (II) 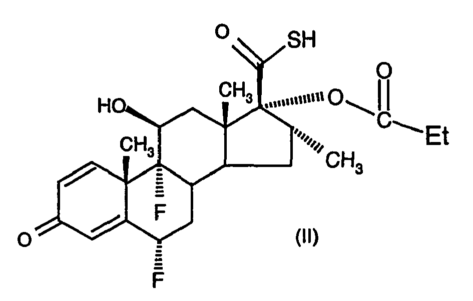 or a salt thereof
which comprises:
(a) reacting a compound of formula (III) 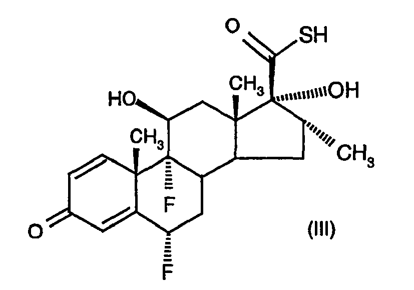 with an activated derivative of propionic acid in an amount of at least 1.3 moles, suitably at least 2 moles, of the activated derivative per mole of compound of formula (III) and;
(b) removal of the sulphur-linked propionyl moiety from any compound of formula (IIA) 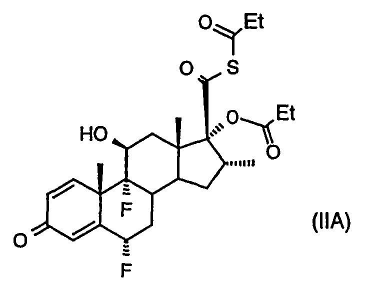 so formed by reaction of the product of step (a) with an organic primary or secondary amine base capable of forming a water soluble propanamide which may then be removed by one or more aqueous washing steps.

In step (a), examples of activated derivatives of propionic acid include activated esters or preferably a propionyl halide such as propionyl chloride. This reaction is conventionally performed in the presence of an unreactive organic base such as a triC₁₋₄alkylamine eg triethylamine, tripropylamine, or tributylamine especially triethylamine, more especially tri-n-propylamine. Solvents for this process include substantially water immiscible organic liquid solvents such as ethyl acetate or methyl acetate or water miscible organic liquid solvents such as acetone, N,N-dimethylformamide or N,N-dimethylacetamide, especially acetone.

In step (b), examples of organic primary or secondary amine base capable of forming a water soluble propanamide include amines which are more polar than diethylamine eg an alcoholamine, eg diethanolamine, or a diamine for example N-methylpiperazine. Preferably, N-methylpiperazine is employed. It may be convenient to dissolve the amine in small volume of an organic solvent such as methanol. The aqueous washing steps may suitably be performed with water or dilute acid eg dilute HCl or acetic acid. In these steps, the more dense aqueous layer may be separated from the organic layer by conventional techniques eg running off under gravity.

Preferably steps (a) and (b) are performed at reduced temperature eg 0-5 °C.

Compound of formula (II) obtained from process (b) may be isolated by conventional techniques. However preferably, and in order to derive maximum benefit, the solvent used in step (a) of this process is a substantially water immiscible organic liquid solvent, more preferably the same solvent as is used in step (a), and even more preferably the same solvent as is used in the main process as well. In this way it is not necessary to isolate the compound of formula (II) in solid form following process (b), although it may be preferable to concentrate the compound of formula (II) in the organic solvent by conventional techniques eg distillation optionally under reduced pressure. Thus the solvent used in step (a) is most preferably ethyl acetate, even more preferably pentan-3-one.

Further general conditions pertaining to the conversion of the compound of formula (III) to the compound of formula (II) and salts thereof and the isolation of the end product will be well known to persons skilled in the art.

If it is intended to isolate the compound of formula (II) (although as noted above a principal feature of the invention overall is that such isolation is rendered unnecessary) it may advantageously be isolated in the form of a solid crystalline salt rather than the free compound of formula (II). The preferred salt is formed with a base such as diisopropylethylamine, triethylamine, 2,6-dimethylpyridine, N-ethylpiperidine or with potassium especially triethylamine. Such salt forms of compound of formula (II) are more stable, more readily filtered and dried and can be isolated in higher purity than the free compound of formula (II). The most preferred salt is the salt formed with triethylamine. The potassium salt is also of interest.

The compound of formula (II) may advantageously be isolated with higher efficiency than by means of prior art processes. For example, the process for the preparation of the compound of formula (II) disclosed in G.H. Phillips et al (1994) J Med Chem 37, 3717-3729 involves the isolation of the product from an acetone/water system. The product so prepared is extremely difficult to filter. In contrast, the compound of formula (II), when prepared in accordance with the process as defined above, is far easier to filter. Furthermore, the process of the present invention may also offer improvements in purity.

One such process comprises treating the organic phase containing the compound of formula (II) with a base as to precipitate the compound of formula (II) in the form of a solid crystalline salt. Example bases include triethylamine, 2,6-dimethylpyridine, N-ethylpiperidine or a basic potassium salt eg potassium hydrogen carbonate.

It is additionally advantageous to prepare and use the compound of formula (III) as an imidazole salt. G.H. Phillips et al (1994) J Med Chem 37, 3717-3729 disclose the preparation of a compound of formula (III) from a compound of formula (IV). However, the physicochemical properties of the compound of formula (III) so prepared result in a product which has a very low speed of filtration. The advantages conferred by the preparation of the imidazole salt of the compound of formula (III) include its properties as an easily prepared and rapidly-filterable, easily handled and stored source of a compound of formula (III), which compound may readily be obtained from the salt by acidification, for example with hydrochloric acid. Furthermore, the compound of formula (III) so derived is of enhanced purity. The imidazole salt of the compound of formula (III) may be prepared, isolated, and stored for subsequent use in the process for the preparation of the compound of formula (II) as described herein. Alternatively, the imidazole salt of the compound of formula (III) may be prepared and used directly as a wet cake in the subsequent conversion to a compound of formula (II) thus avoiding the need to dry the imidazole salt before further reaction.

There is also further provided a process for the preparation of the imidazole salt of the compound of formula (III) which process comprises the reaction of a compound of formula (IV) with carbonyldiimidazole and hydrogen sulphide.

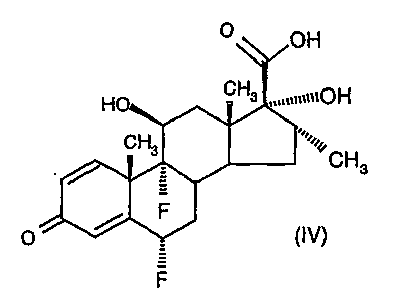

Typically, the compound of formula (IV) and between 1.1 and 2.5 equivalents, suitable 1.8 equivalents, of carbonyldiimidazole are stirred in a suitable solvent, for example ethyl acetate containing between 0 and 2 vol., suitably 0.5 vol., of N,N-dimethylformamide, at a suitable temperature, for example 18-20°C, for a suitable period of time, for example one hour. The resulting suspension is cooled to a suitable temperature, for example -5 to 5°C, suitably -3 to 3°C, and hydrogen sulphide gas introduced over a period of 15-60 minutes, suitably 20-30 minutes, while the suspension is stirred. The reaction mixture is stirred for a further period of about 30 minutes at -5 to 5°C, warmed to about 10°C over a period of about 20 minutes and stirred at 6-12°C for 90-120 minutes. The product is then isolated by filtration, at a suitable temperature, suitable 5-25°C, preferably 10-15°C, washed with a suitable solvent, for example ethyl acetate, and dried *in vacuo* to yield the imidazole salt of the compound of formula (III).

The compound of formula (III) is a monobasic acid and therefore would be expected to form an imidazole salt wherein the stoichiometry of the imidazole salting moiety to the compound of formula (III) is approximately 1:1. However, it has surprisingly been found that the stoichiometry of the imidazole salting moiety to the compound of formula (III) may be up to and including 4:1. Therefore, for the avoidance of doubt, the term "imidazole salt" encompasses imidazole salts of the compound of formula (III) and association compounds of the compound of formula (III) and imidazole wherein the stoichiometry of the imidazole moiety to the compound of formula (III) is up to and including 4:1, for example 1:1 to 4:1, suitably 1.8:1 to 2.5:1. An example of a typical stoichiometry is 2:1. It will be understood that, in the context of stoichiometric values, exact numerical values are to be construed to include nominal variations therefrom.

Preferably, the compound of formula (III) used in the processes described herein is used as an imidazole salt.

It has also been determined to be advantageous to prepare fluticasone propionate directly from the compound of formula (IV) i.e. without the isolation of any intermediate compound. Advantages of such a process include greater efficiency, ease of operation, and the avoidance of handling and storage of chemical intermediates. This process comprises the one-pot preparation of the compound of formula (III) from the compound of formula (IV), followed by *in situ* conversion to the compound of formula (II), followed by the reaction of the compound of formula (II), without isolation (as hereinbefore described) to form fluticasone propionate.

Accordingly, there is provided a process for the preparation of fluticasone propionate, from a compound of formula (IV) without isolation of intermediate compounds, which process comprises the preparation of the compound of formula (III) or a salt thereof from the compound of formula (IV), followed by *in situ* conversion of the compound of formula (III) to the compound of formula (II), followed by *in situ* conversion of the compound of formula (II) to fluticasone propionate.

Typically, suitable solvents, for example a mixture of ethyl acetate and N,N-dimethylformamide are added sequentially to an intimate mixture of the compound of formula (IV) and N,N'-carbonyldiimidazole (6.3g). The resulting suspension is stirred at a suitable temperature, for example 18-20°C for about one hour to give a pale yellow solution. The solution is then cooled to -5 to 25°C, suitably -3 to 3°C and hydrogen sulfide bubbled through the solution over 15-60 minutes, suitably 20-30 minutes maintaining the contents at 12±2°C. The resulting suspension is stirred at 12±2°C for a further 90-120 minutes and then placed under moderate vacuum with a slow nitrogen bleed and stirred vigorously for about one hour. The vacuum is then released and the vessel purged with an inert gas, suitably nitrogen. A suitable solvent, for example 3-pentanone is then added and the slurry washed with aqueous mineral acid, for example 2M hydrochloric acid and then water. The resulting solution is cooled to 0-5°C and tripropylamine added over 2 minutes ensuring that the reaction temperature remains at 0-5°C. The solution is stirred at 0-5°C and propionyl chloride added over about 5 minutes keeping the reaction at 0-5°C. The solution was then allowed to warm to about 10°C and stirred at this temperature for 90-120 minutes. The solution is then cooled to 0-5°C and 1-methylpiperazine added keeping the reaction at 0-5°C. The solution is stirred at 0-5°C for about 20 minutes and then bromofluoromethane added in a single portion. The solution is allowed to warm to ambient temperature and stirred for 16 hours. The solution is then washed sequentially with dilute mineral acid, for example 1M HCl, water, aqueous base, for example 1 % NaHCO₃ and water and then concentrated by atmospheric distillation to about 80ml and then cooled to 90°C. The solution is seeded with fluticasone propionate (Form I) and then a suitable antisolvent, for example toluene added keeping the slurry at about 90°C. The slurry is then concentrated by atmospheric distillation to about 8 vol. The slurry is then cooled to about 90°C. Toluene is then added over 20 minutes keeping the slurry at about 90°C. The slurry is then cooled to about 10°C over 90 minutes, aged at about 10°C for about one hour and then filtered. The cake is washed with a suitable solvent, for example a mixture of 3-pentanone and toluene (1:4) and sucked dry. The solid is then dried by conventional means.

### The invention will be illustrated with reference to the following Examples:

### EXAMPLES

### General

¹H-nmr spectra were recorded at 400MHz and the chemical shifts are expressed in ppm relative to tetramethylsilane. The following abbreviations are used to describe the multiplicities of the signals: s (singlet), d (doublet), t (triplet), q (quartet), m (multiplet), dd (doublet of doublets), ddd (doublet of doublet of doublets), dt (doublet of triplets) and b (broad).

LCMS was conducted on a 25cm X 0.46cm Inertsil ODS-2, 5µm column eluting with 58% {0.1% Formic acid in 3% methanol (aqueous)} (solvent A), and 42% {0.1% Formic acid in 3% methanol (acetonitrile)} (solvent B), using the following elution gradient 0-40 min 42%B, 40-60 min 53%B, 60-75 min 87%B, 75-85 min 42%B at a flow rate of 1 ml/min. The mass spectra were recorded on a HP LC/MSD spectrometer using electrospray positive and negative mode (ES+ve and ES-ve)

Liquid Chromatography (Method A) was conducted on a 25 cm X 0.46 cm ID packed with 5µm Inertsil ODS-2 column eluting with the following acidified mobile phases:
Solution A: Acidified Acetonitrile : Acidified Methanol : Acidified Water (42:3:55)
Solution B: Acidified Acetonitrile : Acidified Methanol : Acidified Water (53:3:44)
Solution C: Acidified Acetonitrile : Acidified Methanol : Acidified Water (87:3:10)
{where acidified acetonitrile comprises of 0.05%v/v Phosphoric acid in acetonitrile (0.5 ml in 1000ml), acidified methanol comprises of 0.05%v/v Phosphoric acid in methanol (0.5 ml in 1000ml) and acidified water comprises of 0.05%v/v Phosphoric acid in water (0.5 ml in 1000ml)}.
The following elution gradient 0-40 min Solution A (100%), 40-60 min Solution B (100 %), 60-75 min Solution C (100%) and 75-90 min Solution A (100%) was run at a flow rate of 1.0 ml / minute at oven temperature of 40°C.

Liquid Chromatography (Method B) was conducted on a Stainless steel 5µm Octyl 20cm x 0.46cm id column eluting with the following acidified mobile phases:
Solution A: Acetonitrile:0.05M aqueous ammonium dihydrogen orthophosphate (35:65) by volume
Solution B: Acetonitrile:0.05M aqueous ammonium dihydrogen orthophosphate (70:30) by volume
The following elution gradient 0-15min Solution A (100%), 15-40 min Solution B (100 %), 40-45 min Solution B (100%) and 45-60 min Solution A (100%) was run at a flow rate of 1.5 ml/minute at oven temperature of 30°C.
Melting points were obtained using Mettler Toledo FP62 melting point apparatus. XRPD's were obtained using a Phillips X'pert MPD powder diffractometer.

### Intermediates

### Intermediate 1: 6α, 9α-difluoro-17α-(1-oxopropoxy)-11β-hydroxy-16α-methyl-3-oxo-androsta-1,4-diene-17β-carbothioic acid

A solution of 6α, 9α-difluoro-11β, 17α-dihydroxy-16α-methyl-3-oxo-androsta-1,4-diene-17β-carbothioic acid (prepared in accordance with the procedure described in GB 2088877B) (7g) in acetone (73.6 ml) is treated at -5°C to -6°C with triethylamine (10.9 ml) over approximately 15 minutes. The solution is stirred at -5°C to 0°C during the addition followed by a wash with acetone (2.8 ml). The resultant suspension is further cooled to -3°C to -7°C and treated with propionyl chloride (6.2 ml) over approximately 30 minutes, maintaining the reaction temperature at -5°C to +2°C. Acetone (2.8 ml) is added as a line wash and the solution is stirred at -5°C to +2°C for a further 2 hours. The resultant suspension is further cooled to -3°C to -7°C and treated with diethanolamine (23.8 ml) in methanol (20 ml) over approximately 30 minutes, maintaining the temperature at -5°C to +2°C. Acetone (2.8 ml) is added as a line wash and the solution is stirred at -5°C to +2°C for a further 30 minutes. The mixture is quenched into water (135 ml) maintaining the temperature at -5°C to +5°C. Acetone (5.6 ml) is added as a line wash and the mixture is cooled to 0°C to 5°C. Concentrated hydrochloric acid (65 ml) is added over one to two hours maintaining the temperature in the range of 0°C to 5°C followed by addition of water (125 ml) maintaining the temperature at <5°C. The mixture is stirred at 0°C to 5°C for 15 minutes, the product is filtered off, washed with water and dried under vacuum at approximately 45°C for 18 hours to give the title compound as a white to off white solid (7.91 g, 99.5 %). HPLC retention time 27.225 min, m/z 469.2 (positive molecular ion) and m/z 467.2 (negative molecular ion).
NMR (DMSO_{d6}) 7.27 (1H, d, 10Hz), 6.34 (1H, d, 10Hz), 6.14 (1H, s), 5.31 (1H, d), 5.17 (1H, ddd), 4.27 (1H, m) 2.40 (2H, q, 7Hz), 2.00-2.14 (5H, m), 1.85 (1H, m), 1.65 (1H, m), 1.51 (3H, s), 1.14 (3H, s), 1.05 (3H, t, 7Hz), 0.88 (3H, d, 7Hz).

### Example 1: 6α. 9α-Difluoro-17α-(1-oxopropoxy)-11β-hydroxy-16α-methyl-3-oxo-androsta-1,4-diene-17β-carbothioic acid S-fluoromethyl ester (using ethyl acetate as solvent and hexane as anti-solvent)

A solution of 6α, 9α-Difluoro-17α-(1-oxopropoxy)-11β-hydroxy-16α-methyl-3-oxo-androsta-1,4-diene-17β-carbothioic acid S-fluoromethyl ester (5.5g) (prepared in accordance with the procedure described by Phillips et al (1994) in J Med Chem, 37, 3717-3729) in ethyl acetate (300 ml) is reduced in volume by vacuum distillation to approximately 32ml and is treated with hexane (63ml) over at least 30 minutes maintaining a temperature of approximately 20°C. The mixture is aged at 20°C for 1 hour and the resultant precipitate is collected by filtration, washed with 1:4 ethyl acetate/hexane (3 x 5 ml) and dried at approximately 50°C for 12 hours to give the title compound as a white solid (5.06g, 92%).
NMR analysis revealed no solvation of the product and XRPD analysis confirmed the product as polymorph Form 1.
HPLC retention time at 35.2 mins.
NMR (DMSO_{d6}) 7.29 (1H, d, 10Hz), 6.34 (1H, d, 10Hz), 6.17 (1H, s), 6.00 (2H, d), 5.68 (1H, ddd), 4.26 (1H, m), 3.40 (3H, s), 2.40 (2H, q, 7Hz), 1.80-2.30 (5H, m), 1.52 (3H, s), 1.14 (3H, s), 1.07 (3H, t, 7Hz), 1.05 (3H, s), 0.94 (3H, d, 7Hz).

### Example 2: 6α, 9α-Difluoro-17α-(1-oxopropoxy)-11β-hydroxy-16α-methyl-3-oxo-androsta-1,4-diene-17β-carbothioic acid S-fluoromethyl ester (using ethyl acetate as solvent and toluene as anti-solvent)

A solution of 6α, 9α-difluoro-17α-(1-oxopropoxy)-11β-hydroxy-16α-methyl-3-oxo-androsta-1,4-diene-17β-carbothioic acid S-fluoromethyl ester (5.5g) (prepared in accordance with the procedure described by Phillips et al (1994) in J Med Chem, 37, 3717-3729) in ethyl acetate (300ml) is reduced in volume by vacuum distillation to 32ml. The reaction mixture is treated with toluene (80ml) and further distilled to a total reaction volume of 32ml. A further portion of toluene (40ml) is added at 60°C and the mixture is cooled to approximately 10°C. The mixture is aged at this temperature for approximately 1 hour and the resultant precipitate is collected by filtration, washed with a mixture of toluene and ethyl acetate (in the ratio 5:1) and dried at approximately 50°C for 12 hours to give the title compound as a white solid (4.70g, 85.4%).
NMR analysis revealed no solvation of the product and XRPD analysis confirmed the product as polymorph Form 1.

### Example 3: 6α, 9α-Difluoro-17α-(1-oxopropoxy)-11β-hydroxy-16α-methyl-3-oxo-androsta-1,4-diene-17β-carbothioic acid S-fluoromethyl ester (using ethyl acetate as solvent and iso-octane as anti-solvent)

A solution of 6α, 9α-diftuoro-17α-(1-oxopropoxy)-11β-hydroxy-16α-methyl-3-oxo-androsta-1,4-diene-17β-carbothioic acid S-fluoromethyl ester 5.5g (prepared in accordance with the procedure described by Phillips et al (1994) in J Med Chem, 37, 3717-3729) in ethyl acetate (300 ml) is distilled to 32ml vol and treated with iso-octane (80 ml) over at least 30 minutes maintaining a temperature of approximately 20°C. The mixture is further distilled to a total reaction volume of approximately 32ml. A further portion of isooctane (40ml) is added at 60°C and the mixture is cooled to approximately 10°C and the mixture aged at this temperature for approximately 60 minutes. The resultant precipitate is collected by filtration, washed with a mixture of ethyl acetate and iso-octane (in the ratio 5 to 1) and dried at approximately 50°C for 12hours to give the title compound as a white solid (5.07g, 92.2% th).
NMR analysis revealed no solvation of the product and XRPD analysis confirmed the product as polymorph Form 1.

### Example 4: 6α, 9α-Difluoro-17α-(1-oxopropoxy)-11β-hydroxy-16α-methyl-3-oxo-androsta-1,4-diene-17β-carbothioic acid S-fluoromethyl ester (using methyl acetate as solvent and toluene as anti-solvent)

A solution of 6α, 9α-diftuoro-17α-(1-oxopropoxy)-11β-hydroxy-16α-methyl-3-oxo-androsta-1,4-diene-17β-carbothioic acid S-fluoromethyl ester (5.5 g) (prepared in accordance with the procedure described by Phillips et al (1994) in J Med Chem, 37, 3717-3729) in methyl acetate (300 ml) is distilled to 32ml and treated with iso-octane (80 ml) over at least 30 minutes maintaining a temperature of approximately 20°C. The mixture is further distilled to a total reaction volume of approximately 32ml. A further portion of toluene (40ml) is added at 60°C and the mixture is cooled to approximately 10°C and the mixture aged at this temperature for approximately 60 minutes. The resultant precipitate is collected by filtration, washed with a mixture of a mixture of methyl acetate and toluene (1:5 ratio) (32ml) and dried at approximately 50°C for 12 hours to give the title compound as a white solid (5.06 gm 92% th).
NMR analysis revealed no solvation of the product and XRPD analysis confirmed the product as polymorph Form 1.

### Example 5: 6α, 9α-Difluoro-17α-(1-oxonropoxy)-11β-hydroxy-16α-methyl-3-oxo-androsta-1,4-diene-17β-carbothioic acid S-fluoromethyl ester (using ethyl acetate as solvent and hexane as anti-solvent)

A solution of 6α, 9α-difluoro-17α-(1-oxopropoxy)-11β-hydroxy-16α-methyl-3-oxo-androsta-1,4-diene-17β-carbothioic acid (Intermediate 1) (3.7g) in ethyl acetate (167ml) and water (13.5 ml) is treated with benzyltributylammonium chloride (0.37g) and triethylamine (1.3 ml) and the mixture is cooled to 5°C. Bromofluoromethane (0.5 ml) is added maintaining a reaction temperature of approximately 5 °C. The mixture is warmed to 20°C over 2-3 hrs and the resultant suspension is sequentially washed with 0.5M hydrochloric acid (23 ml), 1 %w/w aqueous sodium bicarbonate solution (3 x 23 ml) and water (2 x 23 ml). The organic layer is separated and the aqueous layer is optionally back extracted with ethyl acetate (30 ml). The combined organic layers are distilled to an approximate volume of 22 ml and further ethyl acetate (7 ml) is added. The mixture is cooled to approximately 20 °C, hexane (42 ml) is added over at least 30 minutes and the mixture is aged at 20 °C for 15 minutes. The resultant precipitate is collected by filtration, washed with 1:4 ethyl acetate/hexane (3 x 5 ml) and dried at approximately 50°C for 18 hours to give the title compound as a white solid (3.54g, 95.7%w/w). NMR analysis revealed no solvation of the product and XRPD analysis confirmed the product as polymorph Form 1.

This example proved equally efficient for the synthesis of the title compound when repeated using benzyl tributylammonium bromide as phase transfer catalyst or replacing the base with either diisopropylethylamine or sodium bicarbonate.

### Example 6: 6α, 9α-Difluoro-17α-(1-oxopropoxy)-11β-hydroxy-16α-methyl-3-oxo-androsta-1,4-diene-17β-carbothioic acid S-fluoromethyl ester (using ethyl acetate as solvent and toluene as anti-solvent)

A solution of 6α, 9α-difluoro-17α-(1-oxopropoxy)-11β-hydroxy-16α-methyl-3-oxo-androsta-1,4-diene-17β-carbothioic acid (Intermediate 1) (3.7g) in ethyl acetate (167ml) and water (13.5 ml) is treated with benzyltributylammonium chloride (0.37g) and triethylamine (1.3 ml) and the mixture is cooled to 5 °C. Bromofluoromethane (0.5 ml) is added maintaining a reaction temperature of approximately 5 °C. The mixture is warmed to 20°C over 2-3 hrs and the resultant suspension is sequentially washed with 0.5M hydrochloric acid (23 ml), 1 %w/w aqueous sodium bicarbonate solution (3 x 23 ml) and water (2 x 23 ml). The organic layer is separated and the aqueous layer is optionally back extracted with ethyl acetate (30 ml). The combined organic layers are distilled to an approximate volume of 22 ml and toluene (54 ml) is added. The reaction mixture is further distilled to a total reaction volume of approximately 22ml. A further portion of toluene (27ml) is added at approximately 60 °C and the mixture is cooled to approximately 10 °C and aged at this temperature for approximately 1 hour. The resultant precipitate is collected by filtration, washed with a mixture of toluene and ethyl acetate (in the ratio 5:1) and dried at approximately 50°C for 12 hours to give the title compound as a white solid (3.1g, 83.7%). NMR analysis revealed no solvation of the product and XRPD analysis confirmed the product as polymorph Form 1.

### Example 7: 6α, 9α-Ditluoro-17α-(1-oxopropoxy)-11β-hydroxy-16α-methyl-3-oxo-androsta-1,4-diene-17β-carbothioic acid S-fluoromethyl ester ester (using methyl acetate as solvent and iso-octane as anti-solvent)

A solution of 6α, 9α-difluoro-17α-(1-oxopropoxy)-11β-hydroxy-16α-methyl-3-oxo-androsta-1,4-diene-17β-carbothioic acid (3.7g) (Intermediate 1) in ethyl acetate (167ml) and water (13.5 ml) is treated with benzyltributylammonium chloride (0.37g) and triethylamine (1.3 ml) and the mixture is cooled to 5 °C. Bromofluoromethane (0.5 ml) is added maintaining a reaction temperature of approximately 5 °C. The mixture is warmed to 20°C over 2-3 hrs and the resultant suspension is sequentially washed with 0.5M hydrochloric acid (23 ml), 1 %w/w aqueous sodium bicarbonate solution (3 x 23 ml) and water (2 x 23 ml). The organic layer is separated and the aqueous layer is optionally back extracted with ethyl acetate (30 ml). The combined organic layers are distilled to an approximate volume of 22 ml and treated with iso-octane (54ml) over at least 30 minutes maintaining a temperature of approximately 20 °C. The mixture is further distilled to a total reaction volume of approximately 22ml, cooled to approximately 10 °C and aged at this temperature for at least 30 minutes. The resultant precipitate is collected by filtration, washed with a mixture of ethyl acetate and iso-octane (in the ratio 1:5) and dried at approximately 50°C for 12 hours to give the title compound as a white solid (3.4g, 92.3%). NMR analysis revealed no solvation of the product and XRPD analysis confirmed the product as polymorph Form 1.

### Example 8: 6α, 9α-Ditluoro-17α-(1-oxopropoxy)-11β-hydroxy-16α-methyl-3-oxo-androsta-1,4-diene-17β-carbothioic acid S-fluoromethyl ester (using a single solvent to avoid isolation of intermediate 6α, 9α-difluoro-17α-(1-oxopropoxy)-11β-hydroxy-16α-methyl-3-oxo-androsta-1,4-diene-17β-carbothioic acid)

A solution of 6α, 9α-difluoro-11β,17α-dihydroxy-16α-methyl-3-oxo-androsta-1,4-diene-17β-carbothioic acid (prepared in accordance with the procedure described in GB 2088877B) (7g) in ethyl acetate (350 ml) is stirred at 20 to 25 °C and treated at 0 to 5°C with triethylamine (10.9 ml) over approximately 20 minutes adding further ethyl acetate (5 ml) as a line wash. The resultant suspension is further cooled to -3 °C to -7 °C and treated with propionyl chloride (6.2 ml) over approximately 30 minutes, maintaining the temperature at -5 °C to +2 °C. Ethyl acetate (5 ml) is added as a line wash and the solution is stirred at -5 °C to +2 °C for a further 2 hours. The resultant suspension is further cooled to -3°C to -7°C and treated with diethanolamine (23.8 ml) in methanol (20 ml) over approximately 30 minutes, maintaining the temperature at -5 °C to +2 °C. Ethyl acetate (5 ml) is added as a line wash and the solution is stirred at -5°C to +2 °C for a further 30 minutes. Acetic acid (25 ml) is added maintaining the temperature in the range of -5 °C to +2 °C over approximately 10 minutes and the resultant suspension is aged at -5 °C to +5 °C for at least 10 minutes. Water (30 ml) is added over approximately 10 minutes maintaining the temperature in the range of -5°C to +2°C and the organic phase is separated and washed with water (3 x 50 ml). The aqueous phases are optionally back extracted with ethyl acetate (120 ml) at -5 °C to +2 °C and the combined organic phases are concentrated to approximately 45 vol. by vacuum distillation (below 10 °C). Approximately one half of the resultant solution is treated with water (13.5 ml), benzyltributylammonium chloride (0.37g) and triethylamine (1.3 ml) and the mixture is cooled to 5 °C. Bromofluoromethane (0.5 ml) is added maintaining a reaction temperature of approximately 5°C. The mixture is warmed to 20°C over 2-3 hrs and the resultant suspension is sequentially washed with 0.5M hydrochloric acid (23 ml), 1 %w/w aqueous sodium bicarbonate solution (3 x 23 ml) and water (2 x 23 ml). The organic layer is separated and the aqueous layer is back extracted with ethyl acetate (30 ml). The combined organic layers are distilled to an approximate volume of 22 ml and further ethyl acetate (7 ml) is added. The mixture is cooled to approximately 20 °C, hexane (42 ml) is added over at least 30 minutes and the mixture is aged at 20 °C for 15 minutes. The resultant precipitate is collected by filtration, washed with 1:4 ethyl acetate/hexane (3 x 5 ml) and dried at approximately 50 °C for 18 hours to give the title compound as a white solid (3.54g, 95.7%).

The process was successfully repeated when pentan-3-one was employed as solvent in place of ethyl acetate.

### Preparatory Example 9: 6α, 9α-Difluoro-17α-(1-oxopropoxy)-11β-hydroxy-16α-methyl-3-oxo-androsta-1,4-diene-17β-yl S-(1-oxopropoxy) thioanhydride

A solution of 6α, 9α-difluoro-11β,17α-dihydroxy-16α-methyl-3-oxo-androsta-1,4-diene-17β-carbothioic acid (10g) in acetone (125 ml) is cooled to approximately -5 °C and treated at 0 to -5 °C with triethylamine (16 ml) over approximately 15 minutes. The suspension is treated with propionyl chloride (8.5 ml) over approximately 90 minutes, maintaining the temperature at -5 °C to 0 °C and the solution is stirred at -5 °C to 0 °C for a further 2 hours. The reaction mixture is poured into 2M hydrochloric acid (470ml) over ten minutes and the resultant suspension is aged at 5 °C for 30 minutes. The product is filtered off, washed with water (3 x 125ml) and dried under vacuum at approximately 40 °C for 15 hours to give the title compound as a white to off white solid (12.78g, 100.6 %).
HPLC retention time 40.7 mins (99.5area% purity)
CHN: Found C, 61.8%; H, 6.7%; S, 6.1%; C₂₇H₃₄F₂O₆S requires C, 61.8%; H, 6.5%; S, 5.7%.
NMR (DMSO_{d6}) 7.27 (1H, d, 10Hz), 6.32 (1H, d, 10Hz), 6.12 (1H, s), 5.81 (1H, d), 5.65 (1H, ddd), 4.37 (1H, m), 2.40 (2H, q, 7Hz), 2.00-2.45 (4H, m), 1.85 (1H, m), 1.87 (1H, m), 1.51 (3H, s), 1.27 (1H, m), 1.11-1.22 (4H, m), 0.90-1.05 (6H, m), 0.88 (3H, d, 7Hz).

### Preparatory Example 10: Preparation of 6α,9α-difluoro-11β,17α-dihydroxy-16α-methyl-3-oxo-androsta-1,4-diene-17β-carbothioic acid imidazole salt (1:2)

6α,9α-Difluoro-11β-hydroxy-16α-methyl-3-oxo-17α-hydroxy-androsta-1,4-diene-17β-carboxylic acid (35g, 0.089 moles) and carbonyldiimidazole (25.75g, 0.16 moles) are stirred in ethyl acetate (350ml) and N,N-dimethylformamide (17.5ml) at 20 ± 2°C for 60 minutes. The suspension is cooled to 0°C and the batch stirred at 0 ± 5°C whilst hydrogen sulphide (7.7g, 0.23 moles) is added, *via* a sintered glass dip pipe, over 32 minutes. The batch is stirred at 0 ± 3°C for 30 minutes, warmed to 9°C over 20 minutes and stirred at 9± 3°C for a total of 100 minutes. The product is collected by filtration (Whatman 54 paper) and the cake washed with ethyl acetate (2 x 105 ml). The product is dried under vacuum at approximately 20°C for 20 hours to give the title compound as a white to pale purple solid (47.7g, 98.5%th).
NMR (MeOH_{d4}) 0.86 (3H) d, J=7.4 Hz; 1.11 (3H) s; 1.20 (1H) m; 1.61 (3H) s; 1.62-1.82 (3H) m; 2.14-2.25 (2H) m; 2.33 (1H) m; 2.54 (1H) m; 3.19 (1H) m; 4.26 (1H) ddd, J=11.2,4.0,1.8 Hz; 5.57 (1H) dddd, J=49.0,11.6,6.8,1.9 Hz; 6.32 (1H) m; 6.35 (1H) dd, J=10.0,2.0 Hz; 7.35 (4H), d, J=1.0 Hz (imidazole); 7.41 (1H) dd, J=10.0,1.4 Hz; 8.30 (2H) t, J=1.0 Hz (imidazole).
MP 120°C (decomp.)

### Preparatory Example 11: Preparation of a solution of 6α,9α-difluoro-11β,17α-dihydroxy-16α-methyl-3-oxo-ndrosta-1,4-diene-17β-carbothioic acid from 6α,9α-difluoro-11β,17α-dihydroxy-16α-methyl-3-oxo-androsta-1,4-diene-17β-carbothioic acid imidazole salt (1:2)

6α,9α-Difluoro-11β,17α-dihydroxy-16α-methyl-3-oxo-androsta-1,4-diene-17β-carbothioic acid imidazole salt (47.7g) is stirred in ethyl acetate (811 ml) and the suspension is cooled to 15 ± 3°C. 2M (aqueous) hydrochloric acid (286ml) is added and the mixture is stirred for approximately five minutes affording a clear biphasic mixture. The layers are separated and the organic solution of the free carbothioic acid is washed with further 2M hydrochloric acid (190ml).

### Alternative procedure

6α,9α-difluoro-11β,17α-dihydroxy-16α-methyl-3-oxo-androsta-1,4-diene-17β-carbothioic acid imidazole salt (47.7g) is stirred in pentan-3-one (954ml) and the suspension is cooled to 15 ± 3°C. 2M hydrochloric acid (286ml) is added and the mixture is stirred for approximately five minutes affording a clear biphasic mixture. The layers are separated and the organic solution of the free carbothioic acid is washed with water (190ml).

In either case, solvent wet cakes of 6α9α-difluoro-11β17α-dihydroxy-16α-methyl-3-oxo-androsta-1,4-diene-17β-carbothioic acid imidazole salt (rather than dried solids) can be used as inputs to the above acidification procedures.

### Example 12: 6α,9α-Difluoro-17α-(1-oxopropoxy)-11β-hydroxy-16α-methyl-3-oxoandrosta-1,4-diene-17β-carbothioic acid S-fluoromethyl ester

6α, 9α-Difluoro-17α-(1-oxopropoxy)-11β-hydroxy-16α-methyl-3-oxoandrosta-1,4-diene-17β-carbothioic acid imidazole salt (1:2) (13.3g) was suspended in 3-pentanone (200ml) and washed with 2M hydrochloric acid (60ml) and then water (60ml). The resulting solution was cooled to 3°C and tripropylamine (14.0ml) was added over 2 minutes ensuring reaction remained at 3±2°C. The solution was stirred at 3±2°C and propionyl chloride (5.3ml) was added over 5 minutes keeping reaction at 3±2°C. The solution was then allowed to warm to 12°C and stirred at 12±2°C for 90 minutes. The solution was then cooled to 3°C and 1-methylpiperazine (5.1 ml) was added keeping the reaction at 3±2°C. The solution was stirred at 3±2°C for 20 minutes and then bromofluoromethane (1.4ml) was added in a single portion. The solution was allowed to warm to 18°C and stirred at 18±2°C for 16 hours. The solution was then washed sequentially with 1M HCl (60ml), water (60ml), 1% NaHCO₃ (60ml) and water (60ml) and then concentrated by atmospheric distillation to about 80ml and then cooled to 90°C. The solution was seeded with the title compound (0.05g) and then toluene (120ml) was added over 20 minutes keeping the slurry at 87±3°C. The slurry was then concentrated by atmospheric distillation to about 80ml and then cooled to 90°C. Toluene (120ml) was then added over 20 minutes keeping the slurry at 87±3°C. The slurry was then cooled to 10°C over 90 minutes, aged at 10±3°C for 60 minutes and then filtered. The cake was washed with 3-pentanone : toluene (1:4, 2x20ml) and sucked dry. The solid was dried in a vacuum oven at 50°C for 16 hours to give the title compound as a white acicular solid (8.7g, 69%th).

### Example 13: 6α, 9α-Difluoro-17α-(1-oxopropoxy)-11β-hydroxy-16α-methyl-3-oxoandrosta-1,4-diene-17β-carbothioic acid S-fluoromethyl ester

Ethyl acetate (100ml) and N,N-dimethylformamide (5ml) were added sequentially to an intimate mixture of 6α, 9α-difluoro-17α-(1-oxopropoxy)-11β-hydroxy-16α-methyl-3-oxoandrosta-1,4-diene-17β-carboxylic acid (10.0g) and N,N'-carbonyldiimidazole (6.3g). The resulting suspension was stirred at 18-20°C for 50 minutes to give afford a pale yellow solution. The solution was cooled to 10°C and hydrogen sulfide (2.2g) was bubbled through the solution over 25 minutes maintaining the contents at 12±2°C. The resulting suspension was stirred at 12±2°C for a further 90 minutes and then placed under moderate vacuum (300-350mbar) with a slow nitrogen bleed and stirred vigorously for 50 minutes. The vacuum was then released and the vessel was purged with nitrogen. 3-Pentanone (150ml) was then added and the slurry was washed with 2M hydrochloric acid (60ml) and then water (60ml). The resulting solution was cooled to 3°C and tripropylamine (14.0ml) was added over 2 minutes ensuring reaction remained at 3±2°C. The solution was stirred at 3±2°C and propionyl chloride (5.3ml) was added over 5 minutes keeping reaction at 3±2°C. The solution was then allowed to warm to 10°C and stirred at 12±2°C for 90 minutes. The solution was then cooled to 3°C and 1-methylpiperazine (5.1 ml) was added keeping the reaction at 3±2°C. The solution was stirred at 3±2°C for 20 minutes and then bromofluoromethane (1.4ml) was added in a single portion. The solution was allowed to warm to 18°C and stirred at 18±2°C for 16 hours. The solution was then washed sequentially with 1M HCl (60ml), water (60ml), 1% NaHCO₃ (60ml) and water (60ml) and then concentrated by atmospheric distillation to about 80ml and then cooled to 90°C. The solution was seeded with the title compound (0.05g) and then toluene (120ml) was added over 20 minutes keeping the slurry at 87±3°C. The slurry was then concentrated by atmospheric distillation to about 80ml and then cooled to 90°C. Toluene (120ml) was then added over 20 minutes keeping the slurry at 87±3°C. The slurry was then cooled to 10°C over 90 minutes, aged at 10±3°C for 60 minutes and then filtered. The cake was washed with 3-pentanone : toluene (1:4, 2x20ml) and sucked dry. The solid was dried in a vacuum oven at 50°C for 16 hours to give the title compound as a white acicular solid (9.5g, 75%th)

### Example 14: 6α, 9α-Difluoro-17α-(1-oxopropoky)-11β-hydroxy-16α-methyl-3-oxo-androsta-1,4-diene-17β-carbothioic acid S-fluoromethyl ester (using pentan-3-one as solvent and toluene as anti-solvent)

A stirred slurry of 6α, 9α-difluoro-17α-(1-oxopropoxy)-11β-hydroxy-16α-methyl-3-oxoandrosta-1,4-diene-17β-carbothioic acid S-fluoromethyl ester (9.1 g) in pentan-3-one (250ml) was heated to reflux to give a solution which was then concentrated by atmospheric distillation to about 65ml. The solution was cooled to 85°C and then toluene (120ml) was added over 20 minutes maintaining the mixture at 85±3°C. The resulting suspension was concentrated by atmospheric distillation to about 90ml, cooled to 85°C and then toluene (95ml) was added over 20 minutes maintaining the mixture at 85±3°C. The slurry was then aged at 85±3°C for 15 minutes and then cooled to 10°C over 90 minutes. The slurry was aged at 12±3°C for 30 minutes and then filtered. The cake was washed with pentan-3-one : toluene (1:3, 2x20ml) and sucked dry. The solid was dried in a vacuum oven at 50°C for 4 hours to give the title compound as a white acicular solid (9.02g, 98%th). NMR analysis revealed no solvation of the product and XRPD analysis confirmed the product as polymorph Form 1.

## Claims

1. A process for preparing fluticasone propionate as crystalline polymorphic Form 1 which comprises mixing a solution of fluticasone propionate in a non-solvating organic liquid solvent selected from methyl acetate, ethyl acetate and pentanone, with a non-solvating organic liquid anti-solvent selected from toluene, iso-octane and hexane thereby causing fluticasone propionate as crystalline polymorphic Form 1 to crystallise out of the solution, wherein the process is performed at ambient atmospheric pressure.

2. A process according to claim 1 wherein the non-solvating organic liquid solvent is pentan-3-one.

3. A process according to claim 1 or claim 2 wherein the non-solvating organic liquid anti-solvent is toluene.

4. A process according to claim 1, in which the solution of fluticasone propionate in the non-solvating organic liquid solvent is prepared by the following steps:
(a) reacting a compound of formula (II) 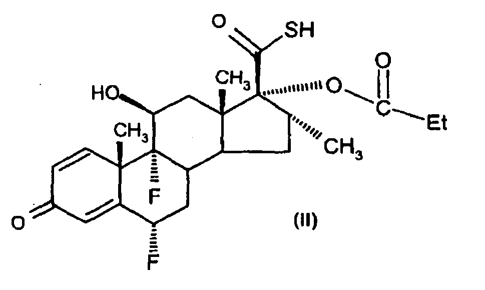 or a salt thereof with a compound of formula LCH₂F wherein L represents leaving group optionally in the presence of a phase transfer catalyst, a water-immiscible non-solvating organic liquid solvent selected from methyl acetate, ethyl acetate and pentanone, and water;
(b) optionally increasing the purity of the fluticasone propionate in the organic layer by performing one or more aqueous washing steps;
(c) separating the organic layer from the aqueous layer and optionally concentrating the fluticasone propionate in the organic layer.

5. A process according to claim 4 wherein L represents halogen.

6. A process according to claim 4 or 5 wherein the water immiscible organic liquid solvent is pentan-3-one.

7. A process according to any one of claims 4 to 6 wherein the compound of formula (II) or a salt thereof is prepared by a process comprising:
(a) reacting a compound of formula (III) 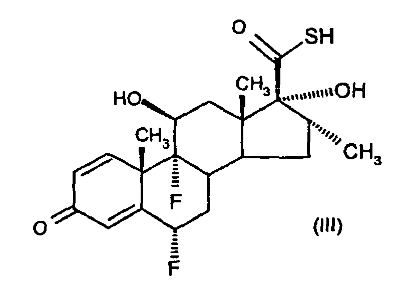 with an activated derivative of propionic acid in an amount of at least 1.3 moles of the activated derivative per mole of compound of formula (III) and;
(b) removal of the sulphur-linked propionyl moiety from any compound of formula (IIA) 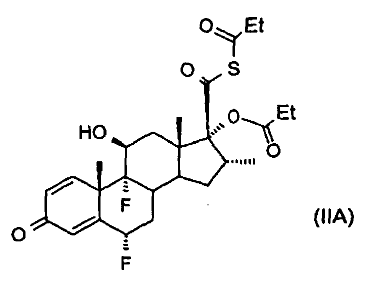
(a) so formed by reaction of the product of step (a) with an organic primary or secondary amine base capable of forming a water soluble propanamide which may then be removed by one or more aqueous washing steps.

8. A process according to claim 7 wherein process (a) is performed in the presence of substantially water immiscible organic liquid solvent.

9. A process according to claim 8 wherein the solvent is pentan-3-one.

10. A process according to any one of claims 7 to 9 wherein the compound of formula (III) is used as an imidazole salt.

11. A process according to any one of claims 7 to 10 wherein the compound of formula (II) is not isolated in solid form.

## Patentansprüche

1. Verfahren zur Herstellung von Fluticasonpropionat als kristallin-polymorphe Form 1, umfassend das Mischen einer Lösung von Fluticasonpropionat in einen nicht-solvatisierenden, organischen, flüssigen Lösungsmittel, ausgewählt aus Methylacetat, Ethylacetat und Pentanon, mit einem nicht-solvatisierenden, organischen, flüssigen Anti-Lösungsmittel, ausgewählt aus Toluol, Isooctan und Hexan, wodurch das Auskristallisieren von Fluticasonpropionat als kristallin-polymorphe Form 1 aus der Lösung bewirkt wird, worin das Verfahren bei Umgebungsdruck durchgeführt wird.

2. Verfahren gemäß Anspruch 1, worin das nicht-solvatisierende, organische, flüssige Lösungsmittel Pentan-3-on ist.

3. Verfahren gemäß Anspruch 1 oder Anspruch 2, worin das nicht-solvatisierende, organische, flüssige Anti-Lösungsmittel Toluol ist.

4. Verfahren gemäß Anspruch 1, worin die Lösung von Fluticasonpropionat in dem nicht-solvatisierenden, organischen, flüssigen Lösungsmittel mittels der folgenden Schritte hergestellt wird:
(a) Umsetzen einer Verbindung der Formel (II) 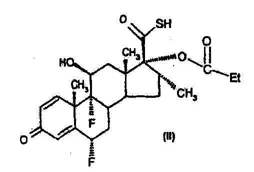 oder eines Salzes davon mit einer Verbindung der Formel LCH₂F, worin L eine Abgangsgruppe darstellt, wahlweise in Gegenwart eines Phasentransferkatalysators, eines mit Wasser nicht mischbaren, nicht-solvatisierenden, organischen, flüssigen Lösungsmittels, ausgewählt aus Methylacetat, Ethylacetat und Pentanon und Wasser;
(b) wahlweise Erhöhen der Reinheit des Fluticasonpropionats in der organischen Schicht mittels Durchführung eines oder mehrerer wässriger Waschschritte;
(c) Abtrennen der organischen Schicht von der wässrigen Schicht und wahlweise Konzentrieren des Fluticasonpropionats in der organischen Schicht.

5. Verfahren gemäß Anspruch 4, worin L Halogen darstellt.

6. Verfahren gemäß Anspruch 4 oder 5, worin das mit Wasser nicht mischbare, organische, flüssige Lösungsmittel Pentan-3-on ist.

7. Verfahren gemäß einem der Ansprüche 4 bis 6, worin die Verbindung der Formel (II) oder ein Salz davon mittels eines Verfahrens hergestellt wird, umfassend:
(a) Umsetzen einer Verbindung der Formel (III) 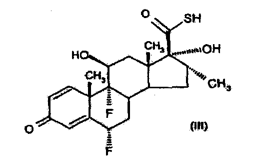 mit einem aktivierten Propionsäurederivat in einer Menge von mindestens 1,3 mol des aktivierten Derivats pro mol der Verbindung der Formel (III) und
(b) Entfernen der Schwefel-verknüpften Propionyleinheit aus einer so gebildeten Verbindung der Formel (IIA) 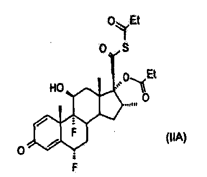 mittels Umsetzung des Produkts aus Schritt (a) mit einer organischen primären oder sekundären Aminbase, die in der Lage ist, ein wasserlösliches Propanamid zu bilden, das anschließend mittels eines oder mehrerer wässriger Waschschritte entfernt werden kann.

8. Verfahren gemäß Anspruch 7, worin das Verfahren (a) in Gegenwart eines im wesentlichen mit Wasser nichtmischbaren, organischen, flüssigen Lösungsmittels durchgeführt wird.

9. Verfahren gemäß Anspruch 8, worin das Lösungsmittel Pentan-3-on ist.

10. Verfahren gemäß einem der Ansprüche 7 bis 9, worin die Verbindung der Formel (III) als Imidazolsalz verwendet wird.

11. Verfahren gemäß einem der Ansprüche 7 bis 10, worin die Verbindung der Formel (II) nicht in fester Form isoliert wird.

## Revendications

1. Procédé de préparation du propionate de fluticasone sous la forme polymorphe cristalline 1, qui comprend le mélange d'une solution de propionate de fluticasone dans un solvant liquide organique non solvatant choisi parmi l'acétate de méthyle, l'acétate d'éthyle et la pentanone, avec un antisolvant liquide organique non solvatant choisi parmi le toluène, l'iso-octane et l'hexane, provoquant ainsi la cristallisation de la forme polymorphe cristalline 1 à partir de la solution, où le procédé est réalisé à la pression atmosphérique ambiante.

2. Procédé selon la revendication 1, dans lequel le solvant liquide organiqus non solvatant est la pentan-3-one.

3. Procédé selon la revendication 1 ou la revendication 2 où l'antisolvant liquide organique non solvatant est le toluène.

4. Procédé selon la revendication 1, dans lequel la solution de propionate de fluticasone dans le solvant liquide organique non solvatant est préparée par les étapes suivantes :
(a) la réaction d'un composé de formule (II) 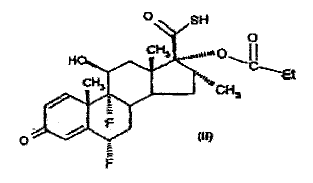 ou d'un sel de celui-ci avec un composé de formule LCH₂F dans laquelle L représente un groupe libérable éventuellement en présence d'un catalyseur de transfert de phase, d'un solvant liquide organique non solvatant non miscible à l'eau choisi parmi l'acétate de méthyle, l'acétate d'éthyle et la pentanone et d'eau ;
(b) l'augmentation éventuelle de la pureté du propionate de fluticasone dans la couche organique en réalisant une ou plusieurs étapes de lavage aqueux ;
(c) la séparation de la couche organique de la couche aqueuse et la concentration éventuelle du propionate de fluticasone dans la couche organique.

5. Procédé selon la revendication 4, dans lequel L représente un atome d'halogène.

6. Procédé selon la revendication 4 ou 5, dans lequel le solvant liquide organique non miscible à l'eau est la pentan-3-one.

7. Procédé selon l'une quelconque des revendications 4 à 6, dans lequel le composé de formule (II) ou un sel de celui-ci est préparé par un procédé comprenant :
(a) la réaction d'un composé de formule (III) 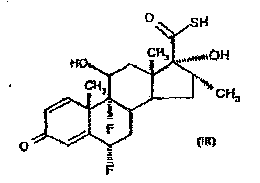 avec un dérivé activé de l'acide propionique dans une quantité d'au moins 1,3 mole du dérivé activé par mole de composé de formule (III) ; et
(b) l'élimination du radical propionyl lié à un atome de soufre de tout composé de formule (IIA) 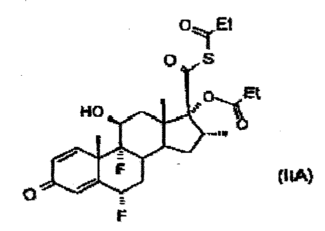 ainsi formé par la réaction du produit de l'étape (a) avec une base organique d'amine primaire ou secondaire capable de former un propanamide soluble dans l'eau qui peut être ensuite éliminé par une ou plusieurs étapes de lavage aqueux.

8. Procédé selon la revendication 7, dans lequel le procédé (a) est réalisé en présence de solvant liquide organique pratiquement non miscible à l'eau.

9. Procédé selon la revendication 8, dans lequel le solvant est la pentan-3-one.

10. Procédé selon l'une quelconque des revendications 7 à 9, dans lequel le composé de formule (III) est utilisé sous la forme d'un sel d'imidazole.

11. Procédé selon l'une quelconque des revendications 7 à 10, dans lequel le composé de formule (II) n'est pas isolé sous forme solide.
